# FASCICULE DE BREVET EUROPEEN

(11) **EP 3 490 523 B1**
(45) Date de publication et mention de la délivrance du brevet: **25.02.2026**
(21) Numéro de dépôt: 17758589.0
(22) Date de dépôt: 27.07.2017
(51) Int. Cl.: A61K 8/37, A61K 8/06, A61K 8/34, A61K 8/73, A61K 8/25, A61Q 1/04

(54) **PRODUIT COSMETIQUE POUR LES LEVRES SOUS LA FORME D'UNE EMULSION EAU-DANS-HUILE ET PROCEDE DE MAQUILLAGE**
KOSMETISCHES PRODUKT FÜR DIE LIPPEN IN FORM VON EINER WASSER-IN-ÖL EMULSION UND SCHMINKVERFAHREN
COSMETIC PRODUCT FOR THE LIPS IN THE FORM OF A WATER-IN-OIL EMULSION AND MAKE-UP PROCESS

(30) Priorité: 28.07.2016 FR 1657296
(43) Date de publication de la demande: 05.06.2019
(73) Titulaire: LvmH Recherche, 45800 Saint-Jean De Braye (FR)
(72) Inventeur: BROSSARD, Fabienne, 45000 Orleans (FR); PHILIPPE, Amandine, 45100 Orléans (FR); DE LA POTERIE, Valérie, 45740 Lailly En Val (FR)
(74) Mandataire: Cabinet Beau de Loménie
(86) Numéro de dépôt international: PCT/FR2017/052101
(87) Numéro de publication internationale: WO 2018/020165

(56) Documents cités:
- EP-A1- 1 457 201
- EP-A1- 2 460 510
- FR-A1- 3 003 465
- US-A1- 2006 019 848
- US-B1- 6 444 212
- "Fluid Stick Lipstick", GNPD, MINTEL, 1 May 2014 (2014-05-01), XP002782677

## Description

La présente invention concerne une composition cosmétique sous forme d'une émulsion eau-dans-huile renfermant un mélange d'huiles particulier, et se rapporte plus particulièrement au domaine du maquillage des lèvres.

Elle concerne également un procédé cosmétique de maquillage des lèvres, comprenant l'application topique sur les lèvres de cette composition.

### Art Antérieur

Les produits de maquillage des lèvres sont des produits cosmétiques pour lesquels les propriétés de brillance sont recherchées contrairement à d'autres produits cosmétiques, comme par exemple les produits de soin de la peau pour lesquels on recherche un film sur la peau au rendu mat le plus discret possible.

Le niveau de tenue d'un produit de maquillage au cours du temps doit également être beaucoup plus élevé sur les lèvres qu'un produit cosmétique appliqué sur d'autres zones du corps, dans la mesure où le produit est soumis à plus de frottements.

Enfin, le confort procuré par le produit doit également être très élevé, car les lèvres sont beaucoup plus sensibles que d'autres parties du corps.

Aussi, pour pouvoir être proposé à la vente, un produit de maquillage des lèvres doit répondre à un cahier des charges complexe comprenant de multiples critères dont : la stabilité du produit au cours du temps, le confort du produit lors de son application sur les lèvres et au cours du temps une fois qu'il a été appliqué, la brillance du film déposé sur les lèvres, et la tenue du film sur les lèvres au cours du temps.

Ces critères sont difficiles à satisfaire simultanément : par exemple, augmenter la tenue du maquillage au cours du temps ou augmenter la brillance du film déposé sur les lèvres conduit très fréquemment à une diminution du confort du produit sur les lèvres qui peut se traduire par un dessèchement des lèvres, des sensations de collant, ou une lourdeur du dépôt sur les lèvres.

La grande majorité des produits de maquillage des lèvres est formulée à partir de corps gras et ne contient pas d'eau. Le brevet US 6 444 212, la demande US 2006/19848 ainsi que la demande EP 1 457 201 en sont l'illustration : les textures sont solides et procurent généralement des rouges à lèvres de bonne tenue.

Les rouges à lèvres ou les gloss contenant de l'eau peuvent être sous la forme d'émulsions solides ou liquides contenant une phase aqueuse et une phase huileuse. Soit la phase aqueuse est dispersée dans une phase huileuse (émulsion eau-dans-huile), soit la phase huileuse est dispersée dans la phase aqueuse (émulsion huile-dans-eau).

Des formulations de rouges à lèvres fluides en émulsion eau-dans-huile ont été proposées notamment dans la demande FR 3003465. La tenue est atteinte grâce à un polymère vinylique à dendrimère carbosiloxane solubilisé dans des huiles siliconées volatiles et non volatiles.

L'émulsion de l'invention est sous la forme d'une émulsion liquide eau-dans-huile, et présente la particularité d'être très fluide par rapport aux émulsions de l'art antérieur de cette catégorie. Les inventeurs ont trouvé de façon inattendue le moyen de formuler une émulsion stable au cours du temps contenant une grande quantité d'huiles et une grande quantité d'eau en utilisant de faibles quantités d'agents stabilisants, lesquels sont généralement nécessaires pour garantir la stabilité d'un système en émulsion dans le temps. L'émulsion de l'invention présente en outre l'avantage de contenir de faibles quantités d'agents de tenue, notamment des composés siliconés phénylés qui peuvent générer des sensations de collant. Les inventeurs ont notamment trouvé que l'association d'une éthylcellulose, d'une hectorite modifiée avec un composé organique et d'esters de polyglycérols permet d'atteindre un tel objectif. Il est en effet très difficile de concilier la stabilité, la fluidité et la texture d'une émulsion pour éviter qu'elle ne génère du collant ou de l'inconfort sur les lèvres.

La fourniture d'une émulsion eau-dans-huile d'une grande fluidité pour le maquillage des lèvres présente de nombreux avantages : elle permet d'obtenir des films sur les lèvres qui procurent une sensation de fraîcheur à l'application du fait de la présence d'eau, ainsi qu'une sensation de légèreté essentiellement liée à leur finesse. Malgré sa fluidité, l'émulsion de l'invention permet d'obtenir de très bonnes propriétés d'hydratation et de brillance. Ceci est d'autant plus surprenant que le film déposé sur les lèvres est fin.

Un des objectifs de l'invention est donc de proposer un produit destiné à une application sur les lèvres qui procure un maquillage des lèvres très hydratant à l'application et dans le temps sans générer de l'inconfort. Un autre de ces objectifs est d'obtenir un dépôt du produit sur les lèvres qui est très fin et laisse une sensation souple, lisse, légère, douce, non grasse et non collante sur les lèvres.

### Description générale de l'invention

Dans la suite du texte les pourcentages sont exprimés en poids par rapport au poids total de l'émulsion de l'invention, sauf mention explicite contraire.

Un au moins des objectifs de l'invention énoncés précédemment est atteint par une émulsion eau-dans-huile, dont la viscosité dynamique à 25°C et pression atmosphérique est de préférence comprise entre 1 500 et 10 000 mPa.s, comprenant une phase aqueuse, des huiles, un ou plusieurs solvants volatils, et une matière colorante.

Cette émulsion répond aux caractéristiques suivantes
- la phase aqueuse représente entre 25 et 45% en poids et comprend de l'eau, au moins un polyol et éventuellement au moins un actif hydratant,
- les huiles représentent entre 25 et 50% en poids et comprennent au moins une première huile choisie parmi les esters d'un polyglycérol, au moins une deuxième huile choisie parmi les monoesters d'alcool gras, et au moins une troisième huile choisie parmi les alcools gras en C10-C26,
- le ou les solvants volatils choisis parmi les huiles siliconées, un monoalcool en C1-C6, et un hydrocarbure, représentent de 10 à 20%, de préférence de 12 à 18% en poids,

Ladite émulsion contient en outre de 0,5 à 10% en poids, de préférence de 2 à 4% en poids d'un polymère soluble ou solubilisé dans ladite troisième huile. Ladite émulsion peut également contenir de 0,05 à 5% en poids, de préférence de 0,1 à 2% en poids d'un composé gélifiant de l'une des huiles ou du mélange des huiles mentionnées précédemment. On entend par « composé gélifiant » ou « composé gélifiant d'huile(s) », un composé qui augmente la viscosité dynamique à 25°C et pression atmosphérique de l'huile ou du mélange d'huiles dans lequel il est ajouté à une teneur de 0,4 à 20% en poids par rapport au poids de l'huile ou du mélange d'huiles concerné.

### Description détaillée de l'invention

Un des aspects de l'invention concerne une émulsion eau-dans-huile comprenant:
- de 25 à 45% en poids d'une phase aqueuse comprenant de l'eau et au moins un polyol,
- de 25 à 50% en poids d'un mélange d'huiles,
- de 10 à 20% en poids d'au moins un solvant volatil choisi parmi les huiles siliconées, un monoalcool en C1-C6, et un hydrocarbure,
- de 0,05 à 5% d'un composé gélifiant au moins une desdites huiles ou gélifiant le mélange desdites huiles d'huile(s),
- une matière colorante,
caractérisée en ce que le mélange d'huiles comprend au moins une première huile choisie parmi les esters d'un polyglycérol, au moins une deuxième huile choisie parmi les monoesters d'alcool gras, et au moins une troisième huile choisie parmi les alcools gras.
- de 0,5 à 10% en poids d'un polymère solubilisé dans la troisième huile, les pourcentages étant exprimés par rapport au poids de l'émulsion.

En particulier :
- l'eau peut représenter de 30 à 40% en poids d'eau et le polyol représenter de 3 à 15% en poids,
- la première huile peut être un mélange comprenant au moins deux esters de polyglycérol et d'acide gras en C16-C20, de préférence non hydroxylés, et représenter de 1 à 15% en poids,
- la deuxième huile peut être un monoester non hydroxylé comprenant au moins une, de préférence deux chaînes alkyle en C16-C20 saturé, et représenter de 1 à 15% en poids,
- la troisième huile peut être un monoalcool saturé en C10-C26, et représenter de 10 à 30 % en poids,
- le solvant volatil peut comprendre au moins 80% en poids d'un solvant siliconé, et
- le composé gélifiant peut être une hectorite modifiée avec un chlorure d'alkylammonium quaternaire, et représenter de 0,1 à 1% en poids,
- de 0,1 à 5% en poids de matière colorante,
les pourcentages étant exprimés en poids par rapport au poids de l'émulsion.

Cette émulsion peut présenter l'avantage d'être à la fois très fluide et stable. Sa viscosité dynamique à 25°C et pression atmosphérique est de préférence comprise entre 1 500 et 10 000 mPa.s.

Un homme du métier saura vérifier sur la base de techniques faisant partie de ses connaissances générales, que la phase aqueuse est dispersée dans une autre phase comprenant lesdites huiles.

La présente demande décrit par ailleurs une émulsion eau-dans-huile de l'invention, dont la viscosité dynamique à 25°C et pression atmosphérique est de préférence comprise entre 1 500 et 10 000 mPa.s, peut comprendre une phase aqueuse, des huiles, un ou plusieurs solvants volatils, et une matière colorante, et être caractérisée en ce que:
- la phase aqueuse représente entre 25 et 45% en poids,
- les huiles représentent entre de 25 et 50% en poids et comprennent au moins une première huile choisie parmi les esters d'un polyglycérol,
- le ou les solvants volatils représentent de 10 à 20% en poids,
ladite émulsion contenant en outre de 1 à 4% en poids d'éthylcellulose, et de 0,05 à 5% en poids d'un composé gélifiant de l'une desdites huiles ou du mélange desdites huiles,
les pourcentages étant exprimés en poids par rapport au poids de l'émulsion.

Un exemple d'émulsion de l'invention est une émulsion eau-dans-huile qui contient :
- de 30 à 40% en poids d'eau,
- de 1 à 15% en poids d'un monoester non hydroxylé comprenant au moins une, de préférence deux chaînes alkyle en C16-C20 saturé,
- de 10 à 30 % d'un monoalcool saturé en C10-C26,
- de 0,1 à 10% en poids d'au moins un polymère solubilisé dans ledit monoalcool,
- de 10 à 20% en poids d'un mélange de solvants volatils comprenant au moins 80% en poids d'un solvant siliconé,
- de 1 à 15% en poids d'un mélange comprenant au moins deux esters de polyglycérol et d'acide gras en C16-C20, de préférence non hydroxylés,
- de 0,1 à 1% en poids d'une hectorite modifiée avec un chlorure d'alkylammonium quaternaire, et
- de 3 à 15% en poids de polyols,
les pourcentages étant exprimés en poids par rapport au poids de l'émulsion.

Dans la présente demande l'expression « de...à ...» vise à comprendre les bornes inférieure et supérieure de la plage de valeurs, tandis que l'expression « entre ... et... » exclut les bornes de la plage de valeurs. La divulgation d'une plage de valeurs excluant ses bornes vaut divulgation de la plage de valeurs équivalente incluant les bornes, et *vice versa.*

Une « huile », au sens de la présente invention, est un composé qui n'est pas soluble à 25°C et pression atmosphérique dans la phase aqueuse de l'émulsion, à la quantité à laquelle la phase aqueuse et l'huile sont utilisées. Un composé « liquide » est une molécule ou un mélange de molécules dont le point de fusion, la température de ramollissement ou le point de transition vitreuse est inférieur ou égal à 30°C, de préférence inférieur ou égal à 25°C, voir même inférieur ou égale au à 20°C, le point de fusion correspondant au maximum de la courbe obtenue par calorimétrie différentielle à balayage, ou au point de fusion commençante de la courbe obtenue par calorimétrie différentielle à balayage. On entend par « solide », un composé qui n'est pas liquide.

Par « acide gras », on entend un acide carboxylique, linéaire ou ramifié, saturé ou insaturé, comprenant de 8 à 30 atomes de carbone, de préférence de 10 à 24 atomes de carbone, encore plus préférentiellement de 12 à 22, et mieux de 16 à 20 atomes de carbone, substitué ou non substitué par un ou plusieurs groupements hydroxyles. Selon un mode de réalisation particulier de l'invention, le ou les acides gras sont linéaires, saturés et non substitués par un groupement hydroxyle.

Dans un mode de réalisation particulier, l'émulsion contient au moins 25% en poids d'un mélange d'huiles, au moins 25% en poids d'eau, de 10 à 20% en poids d'un solvant volatil siliconé, de 0,5 à 10%, de préférence de 2 à 4% en poids d'une éthylcellulose, de 0,05 à 5% en poids d'une hectorite modifiée avec un composé organique, ledit mélange d'huiles comprenant de l'isotéarate d'isostéaryle, au moins un ester de polyglycérol, et de l'octyldodécanol.

L'émulsion de l'invention est de préférence un produit de maquillage des lèvres, c'est-à-dire un produit doté de propriétés particulières qui n'est pas adapté à d'autres utilisations telles qu'un produit de soin de la peau (lotion, sérum, crème), un produit de maquillage de la peau (fond de teint) ou un produit de maquillage des cils (mascara).

Enfin, les derniers aspects de l'invention portent sur i) un procédé de maquillage ou de soin des lèvres qui consiste à appliquer sur les lèvres une des émulsions décrites précédemment, ii) un flacon contenant une des émulsions décrites précédemment.

Chacun des aspects énoncés précédemment peuvent comprendre ou mettre en œuvre un des ingrédients qui sera décrit ci-dessous.

La phase aqueuse représente avantageusement de 25 à 45% en poids, par exemple de 30 à 40% en poids, par rapport au poids de l'émulsion. La phase aqueuse peut contenir de l'eau, un polyol et/ou un sel de sodium ou de magnésium, notamment le chlorure de sodium.

Un polyol peut être incorporé dans la phase aqueuse et comporter au moins deux groupements hydroxyle et de 3 à 8 atomes de carbone. Le polyol peut être par exemple choisi parmi le propylène glycol, le pentylène glycol, la caprylyl glycol, le 1,3 propanediol, le butylène glycol et le glycérol.

L'émulsion de l'invention peut comprendre de 3 % à 15 % en poids de polyol(s), en particulier de 5 % à 10 % en poids de polyol(s) par rapport au poids de l'émulsion.

L'émulsion selon l'invention peut comprendre au moins une première huile choisie parmi les esters de polyglycérol. L'ester de polyglycérol est avantageusement choisi parmi les esters de polyglycérol non oxyalkylénés, par exemple les esters non oxyalkylénés du polyglycérol-2.

L'ester de polyglycérol peut avoir à la fois une fonction émolliente et une fonction de stabilisation de la phase aqueuse dispersée dans la phase grasse.

Un ester de polyglycérol peut être choisi parmi les esters résultant de l'estérification d'un polyglycérol avec un acide polyricinoléique, appelés polyricinoléates de polyglycéryle. A titre d'exemple, on peut citer les polyglycéryl-3 polyricinoléates, les polygycéryl-5 polyricinoléates, les polyglycéryl-6 polyricinoléates, et les polyglycéryl-10 polyricinoléates (dans leurs dénominations INCI). Selon un mode de réalisation particulier de l'invention, on choisit un polyglycéryl-6 polyricinoléate. L'émulsion de l'invention peut comprendre de 1% à 3% en poids d'un composé polyglycéryl polyricinoléate (ou polyricinoléate de polyglycéryle), en particulier de 1,5% à 2% en poids par rapport au poids de l'émulsion.

Les acides gras sont de préférence choisis parmi les acides gras saturés non substitués par un groupement hydroxyle comprenant de 8 à 30 atomes de carbone et éventuellement au moins une ramification méthyle. Les acides gras peuvent être choisis parmi l'acide oléique, l'acide laurique, l'acide palmitique, l'acide myristique, l'acide stéarique, l'acide isostéarique, l'acide linoléique, l'acide caprique, l'acide béhénique, ou leurs mélanges.

Un autre ester de polyglycérol peut être choisi parmi les esters résultant de l'estérification d'un polyglycérol avec un acide gras saturé non substitué par un groupement hydroxyle, comprenant éventuellement au moins une ramification méthyle et éventuellement de 16 à 20 atomes de carbone, de préférence l'acide isostéarique. Les esters de polyglycérol contenus dans l'émulsion sont de préférence choisis parmi les esters non oxyalkylénés du polyglycérol-2. L'ester de polyglycérol est ainsi par exemple le polyglycéryl-2 triisostéarate, le polyglycéryl-2 isostéarate ou le polyglycéryl-2 diisostéarate.

Selon un mode de réalisation particulier de l'invention, la première huile est un mélange comprenant au moins un premier ester choisi parmi les esters d'un polyglycérol et d'un acide carboxylique aliphatique comprenant de 16 à 20 atomes de carbone, et au moins un deuxième ester choisi parmi les polyricinoléates de polyglycéryle. L'ester de polyglycérol et d'acide gras en C16-C20 est notamment choisi parmi le polyglycéryl-2 triisostéarate, le polyglycérol-2-isostéarate et leurs mélanges.

Ainsi, la première huile peut être un mélange du polyglycéryl-2 triisostéarate, du polyglycéryl-2 isostéarate et d'un polyglycéryl-6 polyricinoléate.

La première huile représente de préférence de 1 à 15% en poids par rapport au poids de l'émulsion, plus précisément de 5 à 10% ou de 6 à 8% en poids par rapport au poids de l'émulsion.

L'émulsion de l'invention peut contenir au moins une deuxième huile qui est choisie parmi les monoesters d'acides gras, les acides gras étant définis tels que précédemment. On choisira par exemple les monoesters non hydroxylés comprenant au moins une chaîne alkyle en C16-C20 ramifiée. Ces monoesters peuvent être choisis parmi les esters d'un monoacide carboxylique aliphatique en C16-C20 et d'un monoalcool aliphatique en C16-C20, l'un au moins d'un monoacide ou du monoalcool comprenant au moins une ramification méthyle ou éthyle.

Les monoesters comprennent de préférence une chaîne alkyle ramifiée en C18.

On préfère les esters de l'acide isostéarique. La deuxième huile peut être par exemple choisie parmi les composés myristyl isostearate, butyl isostearate, isostearyl isostearate, stearyl isostearate, isocetyl isostearate, isopropyl isostearate, hydrogenated castor oil isotearate, et 2-ethyl-hexyl isostearate (dans leur dénomination INCI).

Selon un mode de réalisation particulier de l'invention la deuxième huile est l'isostéarate d'isostéaryle.

Le poids de la totalité des esters liquides contenus dans l'émulsion de l'invention est de préférence supérieur à 30%, de préférence supérieur à 35%, de préférence encore supérieur à 40%, du poids de la totalité des huiles de ladite émulsion. Dans un mode de réalisation particulier plus de 90% des huiles esters contenues dans l'émulsion de l'invention sont des esters d'acide gras en C16-C20, de préférence des esters d'acide isostéarique. L'émulsion de l'invention peut comprendre entre 10 et 20%, de préférence entre 14 et 18% en poids, d'huiles dotées d'une fonction ester, par rapport au poids de l'émulsion. Une huile ester additionnelle peut être un ester du 1,1,1-trimethylolpropane et d'un acide gras, par exemple le trimethylolpropane triisostearate.

Une troisième huile peut être choisie parmi les alcools gras aliphatiques en C10-C26, de préférence parmi les monoalcools aliphatiques linéaires ou ramifiés, saturés, en C10-C26, de préférence encore en C16-C20 comme le 2-octyldodécanol.

L'émulsion comprend de préférence entre 15 à 30%, de préférence de 20 et 25% de la troisième huile.

On pourra incorporer dans l'émulsion de l'invention une quantité de polymère qui confère des propriétés filmogènes à un dépôt de l'émulsion sur les lèvres. On préfère un polymère solide à 25°C, que l'on solubilise dans une huile de manière à ce que ce polymère soit solubilisé dans cette huile lorsqu'il a été introduit dans l'émulsion.

Le polymère est soluble ou solubilisé dans au moins une des huiles décrites précédemment ou dans le mélange des huiles décrites précédemment. Le polymère est dit soluble lorsqu'il est solubilisé à une concentration massique comprise entre 5 et 40% en poids par rapport au poids de ladite huile ou dudit mélange des huiles
Avant d'être introduit dans l'émulsion de l'invention, le polymère peut être choisi parmi les polymères solubles à 25°C dans la troisième huile lorsque sa concentration dans la troisième huile est comprise entre 10 et 30% en poids. Le polymère est de préférence hydrocarboné. On entend par hydrocarboné un polymère dépourvu de silicium.

Un tel polymère, avant d'être introduit dans une huile, peut être solide à 25°C et peut être choisi parmi les éthylcelluloses, les résines végétales, et les copolymères de la vinylpyrrolidone.

Les résines végétales, au sens de l'invention couvrent plusieurs composés différents :
- la substance sécrétée par certains végétaux puis récoltée sans transformation chimique ultérieure, appelée exsudat résineux, par exemple un exsudat résineux obtenu notamment par gemmage d'un tronc d'arbre,
- une molécule extraite à partir d'un exsudat résineux, ou un mélange de ces molécules, et
- les produits de réaction chimique d'un exsudat résineux ou d'une molécule qu'il contient.

Les résines végétales lorsqu'elles sont récoltées à partir des végétaux sans transformation chimique peuvent être soit sous forme liquide, soit sous forme solide après avoir été séchées par élimination de l'eau qu'elles peuvent contenir. Les résines végétales sont généralement des liquides très visqueux qui sèchent plus ou moins rapidement lorsqu'ils sont placés au contact de l'air.

Un dérivé d'un exsudat résineux est par exemple un ester de glycérol et de rosine (également appelé glyceryl rosinate ou monoester du glycérol et d'un mélange d'acides gras à longue chaîne dérivés de la rosine, ou glyceryl monorosinate, CAS 65997-13-9, CAS 8050-31-5). La rosine (C₁₅H₂₀O₆; CAS 85026-55-7) est un acide aromatique comprenant un motif glucopyranoside qui est extrait de la colophane, laquelle est obtenue après distillation et séchage d'un exsudat récolté à partir des arbres résineux tels que les pins.

On pourra également utiliser une résine provenant de l'arbre Shorea Robusta (nom INCI Shorea Robusta Resin), également appelée gomme de Damar, qui est un exsudat résineux que l'on récolte sur l'arbre du genre *Shorea* et de l'espère *Robusta.*

Selon un mode de réalisation particulier de l'invention, le polymère est une éthylcellulose.

Le degré de substitution des groupes hydroxyles par motif anhydroglucose de l'éthylcellulose va de préférence de 2,0 à 3,0 (ce qui peut correspondre à un pourcentage massique de groupes éthoxyles compris entre 45,0 et 52,5% par rapport au poids du polymère substitué, selon le grade utilisé).

La masse molaire moyenne de l'éthylcellulose est choisie de préférence de manière à ce que la viscosité d'une solution à 5 % en poids dans un mélange de 80 parts de toluène et de 20 parts d'éthanol à 25°C va de 1 à 400 mPa.s, de préférence de 3 à 250 mPa.s, par exemple de 5 à 20 mPa.s.

Dans un mode de réalisation particulier de l'invention, l'éthylcellulose est dissoute dans une huile ou dans le mélange des huiles de l'émulsion de l'invention, et peut correspondre à un grade vendu sous la désignation commerciale ETHOCEL^{®} Standard 7 Premium, ETHOCEL^{®} 100 ou ETHOCEL^{®} 200 (Dow Chemical Co.), ou sous la désignation commerciale AQUALON^{®} NC (Ashland).

On peut citer, parmi les copolymères de la vinylpyrrolidone, le VP/eicosène et le VP/hexadécène.

La quantité de polymère va par exemple de 0,1 à 10% en poids, de préférence encore de 1 à 5%, voire même de 2 à 4% en par rapport au poids de l'émulsion. Les inventeurs ont en effet constaté qu'à un taux supérieur à 10% un étalement de l'émulsion de l'ordre d'une à quelques centaines de microns est à la fois plus épais et plus collant. A un taux inférieur à 0,1%, les propriétés de brillance et d'hydratation d'un étalement de l'émulsion sur les lèvres sont insuffisantes.

Dans un mode de réalisation particulier, on utilise le mélange d'un polymère comme par exemple une éthylcellulose, et d'une hectorite modifiée par un composé organique tel que par exemple un sel de tétra-alkylammonium, par exemple une hectorite telle que décrite ci-après.

On pourra incorporer dans l'émulsion de l'invention, un ou plusieurs composés gélifiants de l'une des huiles de l'émulsion de l'invention, ou du mélange des huiles contenues dans ladite émulsion. On utilise de préférence de 0,05 à 5%, de préférence de 0,1 à 2%, par exemple de 0,5 à 1% en poids de composé(s) gélifiant(s).

A titre d'exemples de composés gélifiants pouvant être utilisés dans le cadre de l'invention, on peut citer les micas naturels modifiés tel que le fluorosilicate d'aluminium, de magnésium et de potassium; les esters de dextrine et d'acide gras tels que le palmitate de dextrine ou le myristate de dextrine; les tri-esters d'acide gras en C8-C30 et de mono- ou poly-glycéryle tel que le tri(hydroxystéarate) de glycéryle (nom INCI : Trihydroxystearin) ; les argiles organomodifiées qui sont des argiles traitées par des composés choisis notamment parmi les amines quaternaires, les amines tertiaires; les silices pyrogénées traitées en surface par un composé siliconé.

L'émulsion de l'invention peut contenir de 0,1 à 1% en poids d'une hectorite modifiée avec un chlorure d'alkylammonium quaternaire, de préférence un ammonium substitué par au moins, de préférence au moins deux alkyles comprenant de 14 à 20 atomes de carbone. L'alkyle peut être le stéaryle. On citera le composé de nom INCI disteardimonium hectorite dans lequel l'ammonium comprend deux méthyles et deux stéaryles.

L'émulsion de l'invention contient avantageusement le mélange d'une éthylcellulose et d'une hectorite modifiée avec un chlorure d'alkylammonium quaternaire, tel que décrit précédemment.

Le ou les solvants volatils qui sont éventuellement utilisés dans l'émulsion sont de préférence constitués à plus de 65% en poids, de préférence à plus de 75%, voire même à plus de 90% en poids, d'un composé siliconé.

Le solvant volatil peut notamment être choisi parmi les huiles siliconées telles que les diméthicones de viscosité 0,5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges. Selon un mode de réalisation particulier de l'invention le solvant volatil est le décaméthyl cyclopentasiloxane.

Le solvant volatil peut également être un monoalcool en C1-C6 tel que l'éthanol, ou un hydrocarbure tel que l'isododécane, l'isodécane, l'isohexadécane, le n-dodécane (C12) et le n-tétradécane (C14), le mélange undécane-tridécane.

Le ou les solvants volatils présents dans l'émulsion de l'invention peuvent représenter de 5 à 20%, par exemple de 10 à 20%, de préférence de 12 à 18%, en poids par rapport au poids de l'émulsion.

L'émulsion de l'invention contient de préférence moins de 5% en poids d'alcools volatiles et/ou d'hydrocarbures volatiles, tels que de l'éthanol ou de l'isododécane, qui peuvent s'avérer être desséchants pour les lèvres et peuvent diminuer le confort et l'hydratation recherchés dans le cadre de l'invention.

Les inventeurs ont en effet constaté que des solvants volatils tels que l'isododécane et l'éthanol peuvent diminuer l'hydratation des lèvres apportée par l'émulsion, lorsqu'ils sont utilisés dans certaines proportions.

Dans un mode de réalisation particulier, le ou les solvants volatils contenus dans l'émulsion sont constitués d'une cyclométhicone, de préférence le décaméthyl cyclopentasiloxane.

L'émulsion de l'invention peut contenir une ou plusieurs molécules et/ou un ou plusieurs extraits végétaux présentant des propriétés hydratantes, tels que les glycols, en particulier le glycérol ou des polyols naturels et tout autre actif hydratant connu de l'homme de l'art.

L'émulsion de l'invention contient une matière colorante qui est choisie parmi les pigments (insolubles dans les huiles et dans la phase aqueuse) et les colorants (solubles dans les huiles ou dans la phase aqueuse).

Parmi les pigments minéraux, on peut citer, à titre d'exemples le dioxyde de titane, éventuellement traité en surface; les oxydes de fer noir, jaune, rouge et brun; le violet de manganèse; le bleu outremer; l'oxyde de chrome; l'oxyde de chrome hydraté et le bleu ferrique.

Parmi les pigments organiques, on peut citer, par exemple, les pigments D & C Red n° 19 ; D & C Red n° 9 ; D & C Red n° 21 ; D & C orange n° 4 ; D & C orange n° 5 ; D & C Red n° 27 ; D & C Red n° 13 ; D & C Red n° 7 ; D & C Red n° 6 ; D &C Yellow n° 5 ; D & C Red n° 36 ; D & C orange n° 10 ; D & C Yellow n° 6 ; D & C Red n° 30 ; D &C Red n° 3 ; le noir de carbone et les laques à base de carmin de cochenille.

Les pigments nacrés peuvent être choisis notamment parmi les pigments nacrés blancs, tels que le mica recouvert d'oxyde de titane, l'oxychlorure de bismuth ; et les pigments nacrés colorés, tels que le mica titane avec des oxydes de fer, le mica titane avec du bleu ferrique ou de l'oxyde de chrome, le mica titane avec un pigment organique du type précité, ainsi que les pigments à base d'oxychlorure de bismuth.

Parmi les colorants, on pourra citer le caramel, Yellow 5, Acid Blue 9/ Blue 1, Green 5, Green 3 / Fast Green FCF 3, Orange 4, Red 4 / Food Red 1, Yellow 6, Acid Red 33 / Food Red 12, Red 40, le carminé de cochenille (CI 15850, CI 75470), Ext. Violet 2, Red 6-7, Ferrie Ferrocyanide, Ultramarines, Acide Yellow 3 / Yellow 10, Acid Blue 3, Yellow 10. Les colorants liposolubles sont par exemple le rouge Soudan, le D&C Red 17, le D&C Green 6, le bêta-carotène, l'huile de soja, le brun Soudan, le D&C Yellow 11, le D&C Violet 2, le D&C Orange 5, le jaune quinoléine, le rocou.

L'émulsion de l'invention contient par exemple de 0,01 à 10% en poids, par exemple de 0,1 à 1% en poids de matières colorantes, et de 0,2 à 0,8% en poids par rapport au poids total de l'émulsion, dans un mode de réalisation particulier.

Outre les ingrédients décrits précédemment, l'émulsion de l'invention peut comprendre au moins un excipient cosmétiquement ou dermatologiquement acceptable qui peut être choisi parmi des parfums, des phospholipides, des électrolytes, des édulcorants pour masquer l'amertume de certains composés de l'émulsion lorsque celle-ci est appliquée sur les lèvres, des ajusteurs de pH et des conservateurs. Un phospholipide particulier est une léctihine non hydrogénée extraite du soja comprenant 23% en poids de phosphatidylcholine et 97% en poids de phospholipides et de glycolipides.

Les propriétés hydratantes de l'émulsion selon l'invention peuvent être mesurées par cornéométrie. Le cornéomètre mesure la variation de capacitance de la peau au cours du temps. Grâce à une sonde composée de deux électrodes métalliques (or) en forme de peigne, une fine couche isolante sépare les électrodes de l'extrémité de la sonde. Un champ électrique est créé à la surface de la peau. La capacitance du système électrode/peau dépend de la constante diélectrique de la peau qui est en contact avec la sonde si bien que, plus la peau est hydratée, plus la constante diélectrique est importante. L'effet hydratant de l'émulsion à un temps T, peut être exprimé sous la forme du pourcentage d'augmentation entre la capacitance mesurée au temps T sur une zone de peau non traitée et la capacitance mesurée au temps T sur une zone de peau sur laquelle on a appliqué l'émulsion de l'invention.

Le pouvoir hydratant d'un mode de réalisation de l'invention, mesuré par cornéométrie, notamment par le protocole décrit précédemment, peut être supérieur à 60%, de préférence supérieur à 65%, 6 heures après son application.

La viscosité de l'émulsion à 25°C et pression atmosphérique est de préférence comprise entre 1 500 et 10 000 mPa.s, de préférence inférieure à 8 000, 6000 mPa.s ou 5 000 mPa.s, de préférence encore comprise entre 2 500 et 4 000 mPa.s.

Cette viscosité peut être mesurée avec un viscosimètre Rhéolab QC (Anton Paar) doté du logiciel Rheoplus en utilisant un mobile et un temps de mesure adaptés, par exemple dans les conditions suivantes:

| Géométries | Vitesse de rotation (tr/min) | Temps de mesure (min) |
|---|---|---|
| Cylindre coaxial : CC27 | 200 | 3 |
| 4 Pales : ST22 - 4V | 100 | 3 |
| Ailette : ST24-2D-2V-2V | 50 | 3 |
| Ancre : ST22 - 2V | 10 | 7 |

Préalablement à la mesure, on aura placé l'émulsion de l'invention dans un pot de 120 ml (Réf : 102171001, Kola Rond VT3 M120 Blanc Pharm) dans une étuve à 25°C pendant 12 heures minimum. Une fois le mobile plongé dans le pot, le niveau de composition doit atteindre le col du pot.

On vérifie que le mobile est bien choisi en mesurant le pourcentage de déviation des mesures qui sont effectuées toutes les 6 secondes. La valeur de la viscosité de l'émulsion est égale, selon ce protocole, à la moyenne des quinze dernières mesures effectuées par l'appareil pendant le temps de mesure ci-dessus indiqué.

Un film humide d'une émulsion selon l'invention aura avantageusement une épaisseur mesurée à 25°C inférieure ou égale à 65 microns, de préférence inférieure ou égale à 60 microns lorsque l'émulsion est déposée sur une plaque de verre à l'aide d'un étaleur automatique et d'un barreau de 100 µm d'épaisseur.

On peut évaluer la finesse d'un dépôt de l'émulsion en déposant le produit sur une plaque de verre à l'aide d'un étaleur automatique et d'un barreau de 100 µm d'épaisseur. Les étalements sont éventuellement placés pendant 30 minutes à 2 heures dans une enceinte thermique à hygrométrie contrôlée, à 25°C et 50% d'humidité relative.

La mesure de l'épaisseur peut être effectuée immédiatement après l'étalement ou dès la sortie de l'enceinte thermique, par toute méthode connue de l'homme du métier.

Une émulsion de l'invention est avantageusement stable, en ce sens qu'elle ne déphase pas au cours du temps : la phase aqueuse et les huiles ne se séparent pas de manière à former deux phases distinctes que l'on peut distinguer par un examen visuel. La stabilité peut ainsi être vérifiée après stockage de l'émulsion dans une étuve à 50°C, 45°C ou 4°C pendant 1 ou 2 mois.

Une émulsion selon l'un des aspects de l'invention peut répondre à une des caractéristiques suivantes ou à une combinaison de plusieurs de ces caractéristiques :
- comprendre moins de 0,2% en poids, de préférence moins de 0,15% en poids, de préférence encore être dépourvue d'un gélifiant aqueux (c'est-à-dire un composé qui augmente la viscosité de l'eau de plus de 5% à 25°C et pression atmosphérique lorsqu'il est soluble ou dispersible dans l'eau), sans que cela nuise à la stabilité de l'émulsion. Dans ce cas, le produit résultant est moins collant, plus fluide et plus glissant à l'application. Comme gélifiant aqueux, on pourra citer l'hydroxyéthylcellulose, les polymères carboxyvinyliques (nom INCI carbomer), les polysaccharides comme les gommes de xanthane, les gommes de guar, les carraghénanes, les hydroxyméthylcellulloses et leurs mélanges. L'émulsion de l'invention est avantageusement dépourvue de l'un des gélifiants précités.
- comprendre moins de 1% en poids, de préférence moins de 0,5%, voire même être dépourvue de composé(s) gras solides ou de composé(s) gras comprenant une phase solide, tels que des cires ou des composés pâteux. On citera notamment comme composé gras solide, l'acide stéarique, une cire de polyéthylène, une cire synthétique, une cire de paraffine ou une cire microcristalline, l'alcool cétylique, l'alcool stéarylique, l'alcool béhénylique et le stéarate de glycéryle. Ces composés peuvent en effet augmenter l'épaisseur du film de composition déposé sur les lèvres et/ou augmenter la sensation de collant que le film peut générer.
- comprendre moins de 6% en poids, de préférence moins de 1% en poids, de préférence être dépourvue d'un des composés suivants : les organopolysiloxanes élastomériques partiellement ou totalement réticulés, les copolymères polystyrène/copoly(éthylène-propylène) et polystyrène/copoly(éthylène-butylène), les polyisobutènes hydrogénés ou non. En effet, ces composés peuvent laisser sur les lèvres une sensation de gras et/ou de collant, que l'invention cherche précisément à éviter,
- comprendre moins de 5%, de préférence moins de 1% en poids, d'un composé siliconé solide ou liquide non volatil, voir n'en contenir aucun ; parmi les composés siliconés on peut citer des résines siliconées, des élastomères de silicones, des surfactants siliconés, des gommes de silicone ou des huiles siliconées phénylées. Les inventeurs ont notamment trouvé que la présence, dans l'émulsion de l'invention, de composés siliconés comme une résine phénylpropyldimethylsiloxysilicate ou une cetyl dimethicone copolyol conduit à des films déposés sur les lèvres qui sont perçus par les utilisateurs comme étant plus gras, plus épais - et donc davantage perceptibles - et moins confortable au cours du temps. L'émulsion de l'invention contient avantageusement moins de 1%, de préférence 0% en poids de composés siliconés phénylés non volatiles. Malgré la très faible teneur, voir l'absence de composés résines siliconées, de polysaccharides ou de phényl diméthicones, la tenue des propriétés de soin et de maquillage du produit de l'invention, en particulier l'hydratation et la tenue de la brillance, est satisfaisante,
- comprendre moins de 0,5% en poids de filtres solaires organiques, car ces derniers diminuent considérablement les qualités sensorielles de l'émulsion,
- comprendre moins de 8% en poids, de préférence moins de 5% en poids, de préférence être dépourvue d'huile(s) ester(s) choisie(s) parmi les triglycérides issus de matières végétales (huile de ricin par exemple) et le pentaérythrityl tétraisostéarate,
- comprendre moins de 2% en poids, de préférence être dépourvue d'ester(s) de sorbitan, ou d'ester(s) de sucrose tels que le sucrose cocoate ou le sorbitan stearate.

L'invention concerne également un procédé de maquillage des lèvres qui consiste à appliquer sur les lèvres une des émulsions décrites précédemment. Toutes les caractéristiques qui ont été décrites en rapport avec ces émulsions s'appliquent au procédé de maquillage de l'invention.

L'invention concerne un flacon doté d'un moyen d'application contenant l'émulsion décrite précédemment. L'émulsion de l'invention est avantageusement conditionnée dans un flacon (ou bouillotte) doté d'un moyen d'application et d'un capot (ou bouchon). Le moyen d'application peut être un pinceau ou une mousse alvéolée, et être avantageusement fixé au capot. Le pinceau est de préférence plat et son extrémité peut être droite ou arrondie. Le flacon peut avoir une forme de cylindre, de cube ou de parallélépipède. L'applicateur peut avoir différentes formes - cylindrique, oblongue ou plate par exemple - et être éventuellement biseauté pour améliorer la précision de l'application de l'émulsion sur les lèvres.

Un mode de réalisation particulier du procédé de préparation d'une des émulsions décrites précédemment. comprend au moins trois étapes. Dans une première étape on mélange la deuxième et la troisième huile, puis on solubilise le polymère dans ce mélange à une température supérieure ou égale à 60°C. Dans une deuxième étape, on abaisse la température du mélange obtenu à l'issue de la première étape à une valeur comprise entre 30 et 60°C, on ajoute ensuite les autres huiles, le gélifiant d'huile(s) et le(s) solvant(s) volatil(s), puis on abaisse la température du mélange à la température ambiante. Enfin dans une troisième étape, on mélange tous les ingrédients de la phase aqueuse, puis on verse la phase aqueuse dans le mélange obtenu à la deuxième étape. Dans une quatrième étape, les pigments peuvent être ajoutés à l'émulsion ainsi obtenue. Le mélange de tous les ingrédients est avantageusement réalisé sous agitation et à pression atmosphérique.

L'invention est illustrée plus en détail par les exemples suivants.

### Exemples:

On a préparé deux émulsions de compositions suivantes.

| Ingrédients | Exemple 1 | Exemple 2 comparatif |
|---|---|---|
| NOM INCI (en majuscules) et/ou fonction et/ou nom chimique (en minuscules) | % massique | |
| ISOSTEARYL ISOSTEARATE | 9,0 | |
| OCTYLDODECANOL | 22,0 | 21,0 |
| Polymère soluble dans l'octyldodécanol (ETHYLCELLULOSE Ethocel^{®} 7 FP) | 3,0 | 5,0 |
| Esters de Polyglycérol | 6,9 | |
| Solvant volatil | 15,8 | 16,7 |
| PENTAERYTHRITYL TETRAISOSTEARATE | | 8,1 |
| HYDROGENATED POLYISOBUTENE | | 6,7 |
| Gélifiant d'huile | 0,6 | 0,8 |
| LECITHIN | 0,5 | 1 |
| SORBITAN SESQUIOLEATE | | 3,0 |
| Conservateur | qs | qs |
| Anti-oxydant | qs | qs |
| WATER | qsp 100 | qsp 100 |
| GLYCERIN | 5,0 | 5,0 |
| CHONDRUS CRISPUS (CARRAGEENAN) | | 0,2 |
| SODIUM CHLORIDE | 0,2 | 0,2 |
| ALCOHOL | | 1,4 |
| PENTYLENE GLYCOL | 3,0 | 2,0 |
| Pigments | 0,5 | 1,5 |

L'émulsion de l'exemple 1 selon l'invention a été fabriquée en mélangeant les ingrédients dans l'ordre dans lequel ils apparaissent dans le tableau ci-dessus.

Dans une première étape on a mélangé la deuxième et la troisième huile, puis on a solubilisé l'éthylcellulose dans ce mélange à une température de 90°C. Dans une deuxième étape, on a abaissé la température du mélange obtenu à l'issue de la première étape à 50°C, on a ajouté ensuite les autres huiles, le gélifiant d'huile(s) et les solvants volatils, puis on a abaissé la température du mélange à 25°C. Enfin dans une troisième étape on a mélangé tous les ingrédients de la phase aqueuse, puis on a versé la phase aqueuse au mélange obtenu à la deuxième étape. Les pigments ont été ajoutés à l'émulsion ainsi obtenue, dans une quatrième étape. Le mélange de tous les ingrédients a été réalisé sous agitation et à pression atmosphérique.

### • Etude de stabilité

Les émulsions sont placées en étuve à 50°C pendant 1 mois.

Résultat : Le produit de l'exemple 1 selon l'invention était stable tandis que le produit de l'exemple 2 comparatif ne l'était pas.

### • Mesure de la viscosité

La viscosité de l'émulsion de l'exemple 1 selon l'invention et celle d'un gloss pour les lèvres correspondant à un produit de l'art antérieur disponible sur le marché, ont été mesurées avec un viscosimètre Rhéolab QC (Anton Paar) doté du logiciel Rheoplus.

La viscosité de l'Exemple 1 selon l'invention a été mesurée avec le mobile Ailette tournant à 50 tour/min pendant 3 minutes, tandis que la viscosité du produit commercial (Fiche Mintel N°2401385) a été mesurée avec le mobile Ancre tournant à 10 tour/min pendant 7 minutes.

Préalablement à la mesure, chaque composition est versée dans un pot de 120 ml (Réf : 102171001, Kola Rond VT3 M120 Blanc Pharm) puis placée dans une étuve à 25°C pendant 12 heures minimum. Une fois le mobile plongé dans le pot, le niveau de composition atteint le col du pot.

La valeur de la viscosité de l'émulsion est égale à la moyenne des quinze dernières mesures effectuées par l'appareil pendant le temps de mesure indiqué ci-dessus.

Résultat : la viscosité de l'exemple 1 de l'invention était comprise entre 2 500 et4 000 mPa.s, et la viscosité d'un produit commercial de l'art antérieur sous la forme d'une émulsion eau-dans-huile (Fiche Mintel N°2401385) était comprise entre 10.000 et 22.000 mPa.s. L'émulsion de l'invention est beaucoup plus fluide que l'émulsion de l'art antérieur.

### • Pouvoir hydratant

Le pouvoir hydratant de l'Exemple 1 de l'invention a été mesuré au moyen d'un cornéomètre CM 825 (Courage et Khazaka) sur 10 volontaires caucasiens, en réalisant une application sur l'avant-bras (T= 0).

Deux zones de peau de 25 centimètres carrés sont choisies selon une distribution aléatoire sur la face intérieure de l'avant-bras de chaque volontaire. L'émulsion est appliquée sur l'une des deux zones déterminées à raison de 2 mg/cm² de façon « in use », la deuxième zone non traitée sert de témoin.

L'effet hydratant de l'émulsion au temps T=X heures, est égal au pourcentage d'augmentation entre la capacitance mesurée au temps T=X heures sur une zone de peau de l'avant-bras non traitée et la capacitance mesurée au temps T=X heures sur une zone de peau de l'avant-bras sur laquelle on a appliqué l'émulsion de l'Exemple 1 selon l'invention après retrait du film résiduel.

On a suivi le même protocole pour mesurer le pouvoir hydratant d'un produit commercial sous la forme d'une émulsion eau-dans-huile (Fiche Mintel N°2401385).

Résultat : Le pouvoir hydratant de l'Exemple 1 selon l'invention à T=6 heures était de +68%. Le pouvoir hydratant du produit de l'art antérieur à T=8 heures était de +57%. Dans les deux cas, les variations observées étaient significatives (p<0,01).

L'émulsion de l'invention a un pouvoir hydratant bien supérieur à celui de l'émulsion de l'art antérieur.

### • Auto-évaluations in vivo

Le produit de l'exemple 1 de l'invention et un produit commercial de l'art antérieur (Fiche Mintel N°2401385) ont été évalués de façon indépendante par deux panels différents en suivant le même protocole.

Panel pour l'évaluation de l'Exemple 1 de l'invention: 32 femmes volontaires de type caucasien âgées de 21 à 64 ans (moyenne d'âge de 47 ans). Application sur les lèvres une fois par jour pendant une semaine, puis en retouche dans la journée.

Panel pour l'évaluation d'un produit commercial de l'art antérieur (Fiche Mintel N°2401385) : 32 femmes volontaires de type caucasien âgées de 26 à 70 ans. Application sur les lèvres deux fois par jour pendant 4 semaines consécutives.

Chaque femme du panel a évalué elle-même les effets et les propriétés du produit en répondant à un questionnaire à l'issue de la période de test. Les volontaires répondent à un questionnaire en notant : « d'accord », « plutôt d'accord », « plutôt pas d'accord », « pas du tout d'accord ». Pour chaque item, le pourcentage de satisfaction (nombre de réponse « d'accord » et « plutôt d'accord ») est calculé.

Résultats : les résultats obtenus sont reportés dans le tableau ci-dessous.

| Propriété évaluée | **Exemple 1** | Produit commercial de l'art antérieur |
|---|---|---|
| La texture est non collante | 88 | 59 |
| Le film est fin et léger | 91 | 69 |
| Ne se sent pas sur les lèvres (s'oublie) | 94 | 78 |
| Le produit procure une sensation de fraicheur à l'application | 84 | 72 |
| La texture est agréable à porter | 94 | 84 |
| Le produit s'applique facilement | 91 | 81 |
| Le film n'est ni trop fin ni trop épais | 84 | 75 |
| Les lèvres sont souples et douces | 94 | 84 |

Tous les résultats étaient statistiquement significatifs.

Le résultat maquillage obtenu avec l'émulsion de l'Exemple 1 selon l'invention a été ressenti par le panel de volontaires comme plus fin, plus confortable, très agréable à porter et moins collant.

### • Mesure du Collant

L'émulsion de l'Exemple 1 de l'invention est déposée sur une plaque de verre à l'aide d'un étaleur automatique et d'un barreau de 100 µm d'entrefer. Les étalements sont disposés pendant 1h00 dans une enceinte thermique à hygrométrie contrôlée, à 25°C et 50% d'humidité.

A l'aide d'un texturomètre TAXT Plus (Stable Micro Systems), équipé d'un capteur de force de 5 Kg, on applique une pression constante durant un temps déterminé, en utilisant un cylindre inox de 5 mm de diamètre, puis on retire cet outil à une vitesse constante. Durant la phase de retrait, l'émulsion exerce une force de succion sur l'outil, qui est corrélée au collant de l'émulsion.

Pour chaque étalement, 5 mesures ont été réalisées. Le cylindre est systématiquement nettoyé à l'éthanol entre 2 mesures. Les mesures ne sont jamais réalisées au même emplacement sur l'échantillon. Le collant de l'émulsion est équivalent au travail de la force de détachement mesuré durant la phase de retrait de l'outil. Il correspond à l'intégrale de la courbe sous l'axe des temps. Ce travail est exprimé positivement en joules par mètre carré.

Paramètres de mesure :
1) Vitesse d'approche : 0,1 mm/sec
2) Vitesse depuis la détection du contact : 0.1 mm/sec
3) Force / pression : 0.197 N / 0.01 MPa
4) Temps de contact : 3 sec
5) Vitesse de retrait : 0,1 mm/sec

On a reproduit le protocole décrit précédemment en utilisant un produit commercial de l'art antérieur (Fiche Mintel N°1716698).

Résultat : le collant de l'émulsion de l'Exemple 1 selon l'invention était égal à 1.43 +/-0.06 J/m². La valeur de collant du produit de l'art antérieur était égal à 1.79 +/- 0.31 J/m².

### • Mesure de l'épaisseur d'un film de composition.

On a déposé sur une plaque de verre à l'aide d'un étaleur automatique et d'un barreau de 100 µm d'épaisseur soit un étalement de l'émulsion de l'Exemple 1 selon l'invention, soit un étalement d'un produit commercial de l'art antérieur (Fiche Mintel N°2401385).

On a ensuite mesuré l'épaisseur de ces dépôts humides, après les avoir laissés pendant une heure dans une enceinte à 25°C et 50% d'humidité relative.

Résultat : l'épaisseur d'un film de l'émulsion de l'Exemple 1 était de 54,3 microns (écart-type = 4,2), et l'épaisseur du produit commercial était de 69,2 microns (écart-type = 1,5).

Un étalement de l'émulsion de l'invention est beaucoup fin qu'un étalement d'une autre émulsion de l'art antérieur, lorsque les deux produits sont étalés selon le même protocole.

## Revendications

1. Emulsion eau-dans-huile comprenant:
- de 25 à 45% en poids d'une phase aqueuse comprenant de l'eau et au moins un polyol,
- de 25 à 50% en poids d'un mélange d'huiles,
- de 10 à 20% en poids d'au moins un solvant volatil choisi parmi les huiles siliconées, un monoalcool en C1-C6, et un hydrocarbure,
- de 0,05 à 5% d'un composé gélifiant au moins une desdites huiles ou gélifiant le mélange desdites huiles,
- une matière colorante,
**caractérisée en ce que** le mélange d'huiles comprend au moins une première huile choisie parmi les esters d'un polyglycérol, au moins une deuxième huile choisie parmi les monoesters d'alcool gras, et au moins une troisième huile choisie parmi les alcools gras, et **en ce qu'**elle comprend de 0,5 à 10% en poids d'un polymère solubilisé dans la troisième huile,
les pourcentages étant exprimés par rapport au poids de l'émulsion.

2. Emulsion selon la revendication 1, **caractérisée en ce que** la phase aqueuse représente de 30 à 40% en poids par rapport au poids de l'émulsion.

3. Emulsion selon l'une des revendications précédentes, **caractérisée en ce que** la première huile est un mélange comprenant un premier ester choisi parmi les esters d'un polyglycérol et d'un acide carboxylique aliphatique comprenant de 16 à 20 atomes de carbone et d'un deuxième ester choisi parmi les polyricinoléates de polyglycéryle.

4. Emulsion selon l'une des revendications précédentes, **caractérisée en ce que** l'ester de polyglycérol est un ester de polyglycérol et d'acide gras en C16-C20 choisi parmi le polyglycéryl-2 triisostéarate, le polyglycérol-2-isostéarate et leurs mélanges..

5. Emulsion selon l'une des revendications précédentes, **caractérisée en ce que** la deuxième huile est un monoester non hydroxylé comprenant au moins une chaîne alkyle en C16-C20, et qu'il est choisi parmi les composés isostearyl isostearate, isocetyl isostearate, isopropyl isostearate, 2-ethyl-hexyl isostearate.

6. Emulsion selon l'une des revendications précédentes, **caractérisée en ce qu'**elle comprend entre 10 et 20%, de préférence entre 14 et 18% en poids, d'huiles dotées d'une fonction ester, par rapport au poids de l'émulsion.

7. Emulsion selon la revendication 1, **caractérisée en ce que** le solvant volatil une huile siliconée choisie parmi les diméthicones de viscosité 0,5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, et le dodécaméthyl pentasiloxane.

8. Emulsion selon la revendication 1, **caractérisée en ce que** le solvant volatil est un hydrocarbure choisi parmi l'isododécane, l'isodécane, l'isohexadécane, le n-dodécane (C12) et le n-tétradécane (C14), et le mélange undécane-tridécane.

9. Emulsion selon l'une des revendications précédentes, **caractérisée en ce que** la troisième huile est l'octyldodécanol et que le polymère est une éthylcellulose.

10. Emulsion selon l'une des revendications précédentes, **caractérisée en ce que** le gélifiant d'huile est une hectorite modifiée avec un chlorure d'alkylammonium quaternaire et représente de 0,1 à 1% en poids par rapport au poids de l'émulsion.

11. Emulsion selon l'une des revendications précédentes, **caractérisée en ce que** :
- l'eau représente de 30 à 40% en poids et le polyol représente de 3 à 15% en poids,
- la première huile est un mélange comprenant au moins deux esters de polyglycérol et d'acide gras en C16-C20, de préférence non hydroxylés, et représente de 1 à 15% en poids,
- la deuxième huile est un monoester non hydroxylé comprenant au moins une, de préférence deux chaînes alkyle en C16-C20 saturé, et représente de 1 à 15% en poids,
- la troisième huile est un monoalcool saturé en C10-C26, et représente de 10 à 30 % en poids,
- le solvant volatil comprend au moins 80% en poids d'un solvant siliconé,
- le composé gélifiant est une hectorite modifiée avec un chlorure d'alkylammonium quaternaire, et représente de 0,1 à 1% en poids,
- la matière colorante représente de 0,1 à 5% en poids,
les pourcentages étant exprimés en poids par rapport au poids de l'émulsion.

12. Emulsion selon la revendication 11 , **caractérisée en ce qu'**elle comprend moins de 5% en poids d'un composé siliconé solide ou liquide non volatil choisi parmi les résines siliconées, les élastomères de silicones, les surfactants siliconés, les gommes de silicone et les huiles siliconées phénylées.

13. Procédé de maquillage des lèvres qui consiste à appliquer sur les lèvres une émulsion selon l'une des revendications 1 à 12.

14. Flacon doté d'un moyen d'application et d'un capot, lequel flacon contient une émulsion selon l'une des revendications 1 à 13, ledit moyen application étant un pinceau ou une mousse alvéolée.

## Patentansprüche

1. Wasser-in-Öl-Emulsion, umfassend:
- 25 bis 45 Gewichts-% einer wässrigen Phase, umfassend Wasser und mindestens ein Polyol,
- 25 bis 50 Gewichts-% eines Ölgemischs,
- 10 bis 20 Gewichts-% mindestens eines flüchtigen Lösungsmittels, das ausgewählt ist aus silikonhaltigen Ölen, einem einwertigen C1-C6-Alkohol und einem Kohlenwasserstoff,
- 0,05 bis 5 % einer Verbindung, die mindestens eines der Öle geliert oder das Gemisch der Öle geliert,
- einen Farbstoff,
**dadurch gekennzeichnet, dass** das Ölgemisch mindestens ein erstes Öl, ausgewählt aus Estern eines Polyglycerins, mindestens ein zweites Öl, ausgewählt aus Fettalkoholmonoestern, und mindestens ein drittes Öl, ausgewählt aus Fettalkoholen, umfasst, und dass sie 0,5 bis 10 Gewichts-% eines in dem dritten Öl solubilisierten Polymers umfasst,
wobei die Prozentangaben bezogen auf das Gewicht der Emulsion ausgedrückt sind.

2. Emulsion nach Anspruch 1, **dadurch gekennzeichnet, dass** die wässrige Phase 30 bis 40 Gewichts-%, bezogen auf das Gewicht der Emulsion, darstellt.

3. Emulsion nach einem der vorherigen Ansprüche, **dadurch gekennzeichnet, dass** das erste Öl ein Gemisch ist, umfassend einen ersten Ester, der ausgewählt ist aus den Estern eines Polyglycerins und einer aliphatischen Carbonsäure, umfassend 16 bis 20 Kohlenstoffatome, und einen zweiten Ester, der ausgewählt ist aus Polyglycerylpolyricinoleaten.

4. Emulsion nach einem der vorherigen Ansprüche, **dadurch gekennzeichnet, dass** der Polyglycerinester ein C16-C20-Fettsäurepolyglycerinester ist, der ausgewählt ist aus Polyglyceryl-2-triisostearat, Polyglycerin-2-isostearat und Gemischen davon.

5. Emulsion nach einem der vorherigen Ansprüche, **dadurch gekennzeichnet, dass** das zweite Öl ein nicht-hydroxylierter Monoester ist, umfassend mindestens eine C16-C20-Alkylkette und dass es ausgewählt ist aus den Verbindungen Isostearylisostearat, Isocetylisostearat, Isopropylisostearat, 2-Ethylhexylisostearat.

6. Emulsion nach einem der vorherigen Ansprüche, **dadurch gekennzeichnet, dass** sie bezogen auf das Gewicht der Emulsion zwischen 10 und 20 Gewichts-%, vorzugsweise zwischen 14 und 18 Gewichts-%, an Ölen umfasst, die mit einer Esterfunktion dotiert sind.

7. Emulsion nach Anspruch 1, **dadurch gekennzeichnet, dass** das flüchtige Lösungsmittel ein silikonhaltiges Öl ist, das ausgewählt ist aus Dimethiconen mit einer Viskosität von 0,5 und 6 cSt, Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, Octamethyltrisiloxan, Decamethyltetrasiloxan und Dodecamethylpentasiloxan.

8. Emulsion nach Anspruch 1, **dadurch gekennzeichnet, dass** das flüchtige Lösungsmittel ein Kohlenwasserstoff ist, der ausgewählt ist aus Isododekan, Isodecan, Isohexadecan, n-Dodecan (C12) und n-Tetradecan (C14) und dem Gemisch Undecan-Tridecan.

9. Emulsion nach einem der vorherigen Ansprüche, **dadurch gekennzeichnet, dass** das dritte Öl Octyldodecanol ist, und dass das Polymer Ethylcellulose ist.

10. Emulsion nach einem der vorherigen Ansprüche, **dadurch gekennzeichnet, dass** das Ölgeliermittel ein Hectorit modifiziert mit einem quaternären Alkylammoniumchlorid ist, und 0,1 bis 1 Gewichts-%, bezogen auf das Gewicht der Emulsion, darstellt.

11. Emulsion nach einem der vorherigen Ansprüche, **dadurch gekennzeichnet, dass**:
- Wasser 30 bis 40 Gewichts-% darstellt und das Polyol 3 bis 15 Gewichts-% darstellt,
- das erste Öl ein Gemisch ist, das mindestens zwei vorzugsweise nicht hydroxylierte C16-C20-Fettsäure- und Polyglycerinester umfasst, und 1 bis 15 Gewichts-% darstellt,
- das zweite Öl ein nicht-hydroxylierter Monoester ist, umfassend mindestens eine, vorzugsweise zwei gesättigte C16-C20-Alkylketten, und der 1 bis 15 Gewichts-% darstellt,
- das dritte Öl ist ein gesättigter einwertiger C10-C26-Alkohol ist und 10 bis 30 Gewichts-% darstellt,
- das flüchtige Lösungsmittel mindestens 80 Gewichts-% eines silikonhaltigen Lösungsmittels umfasst,
- die gelierende Verbindung ein Hectorit ist, das mit einem quaternären Alkylammoniumchlorid modifiziert ist, und 0,1 bis 1 Gewichts-% darstellt,
- der Farbstoff 0,1 bis 5 Gewichts-% darstellt,
wobei die Prozentangaben in Gewicht bezogen auf das Gewicht der Emulsion ausgedrückt sind.

12. Emulsion nach Anspruch 11, **dadurch gekennzeichnet, dass** sie weniger als 5 Gewichts-% einer festen oder flüssigen, nicht flüchtigen Silikonverbindung umfasst, die ausgewählt ist aus Silikonharzen, Silikonelastomeren, Silikontensiden, Silikongummis und phenylierten Silikonölen.

13. Verfahren zum Schminken der Lippen, das darin besteht, eine Emulsion nach einem der Ansprüche 1 bis 12 auf die Lippen aufzutragen.

14. Flasche, die mit einer Auftragseinrichtung und einer Kappe versehen ist, wobei die Flasche eine Emulsion nach einem der Ansprüche 1 bis 13 enthält, wobei die Auftragseinrichtung ein Pinsel oder ein Schaumstoff mit Noppen ist.

## Claims

1. Water-in-oil emulsion comprising:
- from 25 to 45% by weight of an aqueous phase comprising water and at at least one polyol,
- from 25 to 50% by weight of a mixture of oils,
- from 10 to 20% by weight of at least one volatile solvent chosen from silicone oils, a C1-C6 monohydric alcohol, and a hydrocarbon,
- 0.05 to 5% of a gelling compound at least one of said oils or gelling the mixture of said oils,
- a colouring agent,
**characterised in that** the oil mixture comprises at least a first oil selected from polyglycerol esters, at least a second oil selected from fatty alcohol monoesters, and at least a third oil selected from fatty alcohols, and **in that** it comprises from 0.5 to 10% by weight of a polymer solubilised in the third oil,
the percentages being expressed relative to the weight of the emulsion.

2. Emulsion according to claim 1, **characterised in that** the aqueous phase represents 30 to 40% by weight relative to the weight of the emulsion.

3. Emulsion according to one of the preceding claims, **characterised in that** the first oil is a mixture comprising a first ester selected from esters of a polyglycerol and an aliphatic carboxylic acid comprising 16 to 20 carbon atoms and a second ester selected from polyglyceryl polyricinoleates.

4. Emulsion according to one of the preceding claims, **characterised in that** the polyglycerol ester is an ester of polyglycerol and a C16-C20 fatty acid chosen from polyglycerol-2 triisostearate, polyglycerol-2 isostearate and mixtures thereof.

5. Emulsion according to one of the preceding claims, **characterised in that** the second oil is a non-hydroxylated monoester comprising at least one C16-C20 alkyl chain, that is selected from isostearyl isostearate, isocetyl isostearate, isopropyl isostearate and 2-ethylhexyl isostearate.

6. Emulsion according to one of the preceding claims, **characterised in that** it comprises between 10 and 20%, preferably between 14 and 18% by weight, of oils having an ester function, relative to the weight of the emulsion.

7. Emulsion according to claim 1, **characterised in that** the volatile solvent is a silicone oil selected from dimethicones with a viscosity of 0.5 and 6 cst, octamethyl cyclotetrasiloxane, decamethyl cyclopentasiloxane, dodecamethyl cyclohexasiloxane, heptamethyl hexyltrisiloxane, heptamethyloctyl trisiloxane, hexamethyl disiloxane, octamethyl trisiloxane, decamethyl tetrasiloxane, and dodecamethyl pentasiloxane.

8. Emulsion according to claim 1, **characterised in that** the volatile solvent is a hydrocarbon selected from isododecane, isodecane, isohexadecane, n-dodecane (C12) and n-tetradecane (C14), and the undecane-tridecane mixture.

9. Emulsion according to one of the preceding claims, **characterised in that** the third oil is octyldodecanol and the polymer is ethylcellulose.

10. Emulsion according to one of the preceding claims, **characterised in that** the oil gelling agent is a hectorite modified with an alkylammonium chloride and represents from 0.1 to 1% by weight relative to the weight of the emulsion.

11. Emulsion according to one of the preceding claims, **characterised in that**
- water accounts for 30 to 40% by weight and polyol accounts for 3 to 15% by weight,
- the first oil is a mixture comprising at least two polyglycerol esters of C16-C20 fatty acids, preferably non-hydroxylated, and represents 1 to 15% by weight,
- the second oil is a non-hydroxylated monoester comprising at least one, preferably two saturated C16-C20 alkyl chains, and represents 1 to 15% by weight,
- the third oil is a saturated C10-C26 monoalcohol, and represents 10 to 30% by weight,
- the volatile solvent comprises at least 80% by weight of a silicone solvent,
- the gelling compound is a hectorite modified with a quaternary alkylammonium chloride, and represents 0.1 to 1% by weight,
- the colouring agent represents from 0.1 to 5% by weight,
the percentages being expressed by weight relative to the weight of the emulsion.

12. Emulsion according to claim 11, **characterised in that** it comprises less than 5% by weight of a non-volatile solid or liquid silicone compound chosen from silicone resins, silicone elastomers, silicone surfactants, silicone gums and phenylated silicone oils.

13. Method for applying lip make-up, which consists of applying an emulsion according to one of claims 1 to 12 to the lips.

14. Bottle equipped with an application means and a cap, which bottle contains an emulsion according to one of claims 1 to 13, said application means being a brush or a honeycomb foam.
